# EUROPEAN PATENT SPECIFICATION

(11) **EP 1 434 870 B1**
(45) Date of publication and mention of the grant of the patent: **27.12.2006**
(21) Application number: 02779375.1
(22) Date of filing: 19.09.2002
(51) Int. Cl.: C12P 13/04, C12P 11/00, C07C 323/63

(54) **ENZYMATIC PROCESS FOR THE PREPARATION OF SUBSTITUTED 2-AMINO-3-(2-AMINO-PHENYLSULFANYL)-PROPIONIC ACID**
ENZYMATISCHES VERFAHREN ZUR HERSTELLUNG VON SUBSTITUIERTER 2-AMINO-3-(2-AMINO-PHENYLSULFANYL)-PROPIONSÄURE
PROCEDE ENZYMATIQUE DE PREPARATION D'ACIDE 2-AMINO-3-(2-AMINO-PHENYLSULFANYL)-PROPIONIQUE A SUBSTITUTION

(30) Priority: 25.09.2001 EP 01122906
(43) Date of publication of application: 07.07.2004
(73) Proprietor: F. HOFFMANN-LA ROCHE AG, 4070 Basel (CH)
(72) Inventor: BLEICHER, Konrad, 79102 Freiburg (DE); BORTHWICK, Scott, Selkirkshire TD1 1NW (GB); IDING, Hans, 79618 Rheinfelden (DE); ROGERS-EVANS, Mark, CH-4102 Binningen (CH); SCHMID, Stefan, CH-4058 Basle (CH); TONG, Han, Min, F-92160 Antony (FR); WIRZ, Beat, CH-4153 Reinach (CH)
(74) Representative: Schager, Frank
(86) International application number: PCT/EP2002/010511
(87) International publication number: WO 2003/029477

(56) References cited:
- EP-A- 0 178 553
- EP-A- 0 407 033
- EP-A- 0 560 408
- EP-A- 0 577 253
- DE-A- 2 804 892
- DE-A- 4 009 891

## Description

The present invention relates to a new enzymatic process for the preparation of compounds of formula I 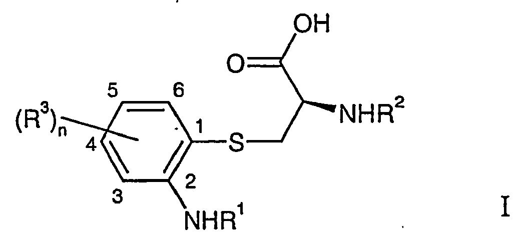 wherein
R¹ is hydrogen or alkyl;
R² is an amino protecting group;
each R³ is independently halogen, carboxyl, alkoxycarbonyl, alkenyloxycarbonyl or benzyloxycarbonyl;
n is 1 or 2.

In EP 0407033 A an enzymatic stereoselective hydrolysis of racemic mixtures of esters of 2-substituted acids, other than 2-halo propionic acids, into the corresponding enantiomeric acids is described. The reaction is carried out in the presence of Candida rugosa lipase isoenzymes, an organic solvent (e.g. toluene) and a reducing agent. The process is especially useful for the stereoselective production of S-ketoprofen, S-ibuprofen, S-fenoprofen, S-2-phenylpropionic acid and S-indoprofen.

In EP 0178553 A the preparation of aromatically substituted L-amino acids by selective hydrolysis of the corresponding alkyl esters with chymotrypsin is described.

In DE 2804892 the preparation of optically pure N-acyl-L-threonine by hydrolysing N-acyl-DL-threonine ester with serine protease, especially subtilisin Carlsberg, is described.

No enzymatic reaction for the preparation of compounds of the formula I has been described in the literature.

Attempts to synthesise compounds of formula I by chemical hydrolysis resulted in low yields and decomposition of the starting material.

The aim of the present invention therefore is a novel and inventive process for the preparation of substituted 2-amino-3-(2-amino-phenylsulfanyl)-propionic acids.

The compounds of formula I may be manufactured by the process of the present invention, which provides a new process for the preparation of substituted 2-amino-3-(2-amino-phenylsulfanyl)-propionic acid of formula I 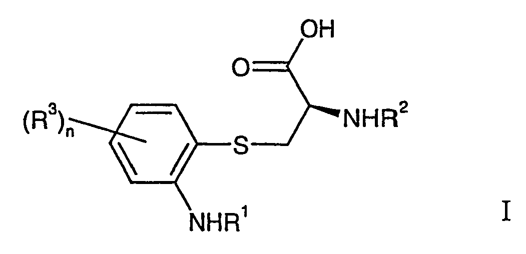 wherein
R¹ is hydrogen or alkyl;
R² is an amino protecting group;
each R³ is independently halogen, carboxyl, alkoxycarbonyl, alkenyloxycarbonyl or benzyloxycarbonyl;
n is 1 or 2,
which process comprises
reacting compounds of formula II 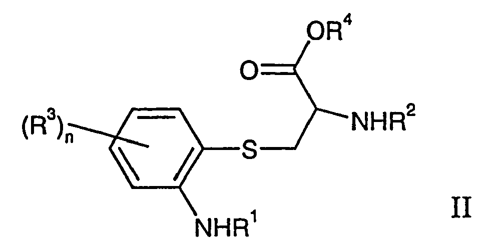 wherein R¹, R², R³ and n are as defined above and
R⁴ is alkyl or benzyl,
with a protease in an aqueous system containing an organic co-solvent.

With the enzymatic approach described herein a selective hydrolysis of the ester under mild reaction conditions is possible.

In the structural formula presented herein a wedged bond ( 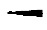 ) denotes that the substituent is above the plane of the paper.

The term "alkyl" as used herein denotes an optionally substituted straight or branched chain hydrocarbon residue containing 1 to 12 carbon atoms, such as methyl, ethyl, n-propyl, isopropyl, n-butyl, sec-butyl, isobutyl, tert.-butyl, pentyl, hexyl, heptyl, octyl, nonyl, decyl, undecyl, dodecyl including their different isomers.

Suitable substituents for the alkyl chain may be selected from 1-3 halogens such as fluorine or chlorine, or C₁₋₄-alkoxy such as methoxy or ethoxy. Examples for substituted alkyl are 2-chloroethyl, 2-fluoroethyl, 2,2,2-trifluoroethyl or 2-methoxyethyl.

Alkyl in R¹ is as defined above and preferably a straight or branched chain hydrocarbon residue containing 1 to 7 carbon atoms. Alkyl in R¹ is more preferred methyl, ethyl, propyl, isopropyl, n-butyl, sec-butyl, isobutyl or tert.-butyl.

Alkyl in R⁴ is as defined above and preferably an optionally substituted straight or branched chain hydrocarbon residue containing 1 to 7 carbon atoms. Suitable substituents for the alkyl chain may be selected from 1-3 halogen such as fluorine or chlorine, or C₁₋₄-alkoxy such as methoxy or ethoxy. Examples for substituted alkyl are 2-chloroethyl, 2-fluoroethyl, 2,2,2-trifluoroethyl or 2-methoxyethyl. Alkyl in R⁴ is more preferred a straight chain hydrocarbon residue containing 1 to 7 or 1 to 4 carbon atoms. Examples are methyl, ethyl, propyl, n-butyl, n-pentyl, n-hexyl, n-heptyl. Preferred examples are methyl, ethyl or propyl. Most preferred alkyl in R⁴ is methyl.

The term "amino protecting group" as used herein refers to groups such as those employed in peptide chemistry as described in Green T. Protective Groups in Organic Synthesis, Chapter 5, John Wiley and Sons, Inc. (1981), pp. 218-287, such as an allyloxycarbonyl group (ALLOC), a lower alkoxycarbonyl group (e.g. tert.-butoxycarbonyl (t-BOC)), a substituted lower alkoxycarbonyl group (e.g. trichloroethoxycarbonyl), an optionally substituted aryloxycarbonyl group (e.g. p-nitrobenzyloxycarbonyl, benzyloxycarbonyl (Z) or phenyloxycarbonyl), an alkanoyl group (e.g. formyl, acetyl), an aroyl group (e.g. benzoyl), a halogen-alkanoyl group (e.g. trifluoroacetyl) or a silyl protective group (e.g. tert.-butyldimethylsilyl). Preferred amino protecting groups are benzyloxycarbonyl, tert.-butoxycarbonyl, allyloxycarbonyl or benzoyl, especially preferred amino protecting group is tert.-butoxycarbonyl.

The term "alkoxycarbonyl" denotes an C₁₋₇-alkoxy residue attached to a carbonyl group (>C=O). Examples for alkoxy groups are methoxy, ethoxy, n-propyloxy, iso-propyloxy, n-butyloxy, 1-sec-butyloxy, iso-butyloxy, tert.-butyloxy, pentyloxy, hexyloxy, heptyloxy including their different isomers. Examples for alkoxycarbonyl groups are methoxycarbonyl, ethoxycarbonyl, propoxycarbonyl, tert.-butoxycarbonyl and the like. Preferred lower alkoxycarbonyl is tert.-butoxycarbonyl.

The term "alkenyloxycarbonyl" denotes an alkenyl-oxy residue attached to an carbonyl group (>C=O). The term "alkenyl" as used herein denotes an unsubstituted or substituted hydrocarbon chain radical having from 2 to 8 carbon atoms, preferably from 2 to 4 carbon atoms, and having at least one olefinic double bond, including their different isomers. Examples are vinyl, allyl or isopropenyl.

Preferred "alkenyloxycarbonyl" for R³ is allyloxycarbonyl or isopropenyloxycarbonyl, more preferred is allyloxycarbonyl.

The term halogen signifies fluorine, chlorine, bromine or iodine. Preferred halogen is bromine.

The number n may be either 1 or 2, preferably the number n is 1.

The substituent R³ may be in any possible position attached to the phenyl ring. If n is 1, R³ may be in the 3,4,5 or 6-position of the phenyl ring. Preferably, the R³ substituent is in the 4-position. If n is 2, both R³ substituents may be in the independently from each other in the 3,4,5 or 6-position of the phenyl ring.

A preferred embodiment of the invention is a process for the preparation of compounds of formula I, characterized in that compound of formula II is 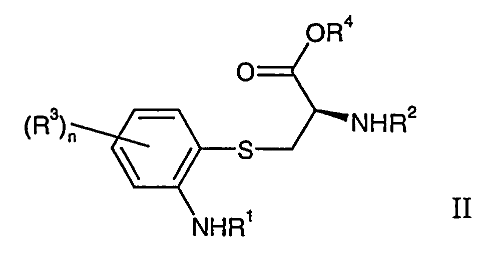 wherein R¹, R², R³, R⁴ and n are as defined above.

In a further preferred embodiment of the process invention
R¹ is hydrogen or alkyl,
more preferred
R¹ is hydrogen;
R² is an amino protecting group,
more preferred
R² is an amino protecting group;
each R³ is independently halogen, carboxyl or alkenyloxycarbonyl;
R⁴ is alkyl or benzyl,
more preferred
R⁴ is alkyl;
n is 1 or 2,
more preferred
n is 1.

In another preferred embodiment of the invention the process is carried out with a protease in an aqueous system, containing an organic co-solvent, at a pH of 4.0 - 10, preferred 6.0 - 8.5.

After the enzymatic hydrolysis of the substrate of formula II the enantiomeric pure product of formula I is separated by acidification and subsequent extraction.

Compound of formula II may be used in any possible mixture of the L or D enantiomers. The conversion is accomplished preferably with a racemic mixture or with a mixture which only contains the L-isomer.

When employing a mixture of isomers, the unreacted remaining D-ester is removed by an extraction step prior to acidification of the reaction medium and subsequent extraction of the L-acid.

Suitable enzymes as catalysts for the reactions are proteases, preferably cheap bulk proteases of microbial origin, more preferred are Bacillus proteases (like Savinase from Novo Nordisk) or subtilisins e.g. subtilisin Carlsberg from Novo Nordisk (Alkalase) or from Solvay (Protease-L) or Aspergillus proteases (like Prozyme 6 from Amano) or Tritachium proteases (Proteinase K from Fluka). Most preferred enzyme as catalyst for the reactions is subtilisin Carlsberg (e.g. Alcalase from Novo Nordisk).

As an alternative the enzymes may be used in immobilized form.

The reaction is carried out in an aqueous system with an organic co-solvent, such as a water-immiscible solvent or a water-miscible organic co-solvent. The water-immiscible solvent may be used in any ratio with the aqueous phase, preferred ratio is 25-75% (v/v). The water-miscible organic co-solvent may be used in an amount as high as tolerated by the enzyme, typically 5-25% (v/v) but which might exceed even 50% (v/v), e.g. in case of subtilisin Carlsberg.

The reaction is carried out at a reaction temperature from 0°C to 50°C, preferred at a reaction temperature between 15°C and 40°C, and most preferred at a reaction temperature between 15°C and 25°C.

As to the aqueous phase, common buffer solutions known in the art for biochemical conversions may be used, such as sodium or potassium phosphate in a concentration of up to 1M, preferably between about 5mM and about 50mM. Such a buffer solution may additionally contain one of the usual salts like e.g. sodium or potassium chloride, and also LiSCN, Na₂SO₄ or a polyhydric alcohol e.g. a sugar; in a concentration up to 1M.

Suitable organic co-solvents are technically common solvents. Examples are ethers (e.g. tetrahydrofuran (THF), dioxan or tert.-butyl methyl ether (TBME)), lower alcohols, esters (e.g. ethyl acetate), polar aprotic solvents (e.g. dimethylsulfoxide (DMSO), dimethylacetamide, N,N-dimethylformamide (DMF) or acetone). Preferred organic co-solvents are tetrahydrofuran (THF), tert.-butyl methyl ether (TBME) and ethyl acetate.

The term "lower alcohol" as used herein denotes straight chain or branched alkyl residues containing 1 to 8 carbon atoms with one hydroxy group, such as methanol, ethanol, propanol, isopropanol, butanol, isobutanol, tert.-butanol, pentanol, hexanol, heptanol or octanol, preferably methanol, ethanol, propanol, isopropanol, butanol, isobutanol, tert.-butanol and more preferred alcohol are methanol or ethanol.

The substrate is suitably applied as a solution in a 0.1- 25% overall concentration (w/w). A more preferred overall concentration is 1-10%.

After addition of the enzyme, the pH of the reaction mixture is maintained under vigorous stirring at the selected pH-value by the controlled addition of a base. Preferred bases are aqueous NaOH or KOH solutions.

After termination of the reaction, the enantiomerically pure product of formula I is worked up conventionally after phase separation, by acidification of the aqueous phase with a suitable acid and subsequent extraction with a suitable organic solvent.

Compounds of formula I are synthesized, according to the present invention, in high enantiomeric purity, which means that enantiomeric excesses of 90% or higher, preferred an enantiomeric excess of 95% or higher may be achieved.

Compounds of formula II are prepared according the following reaction scheme or in a conventional manner known to the skilled in the art. 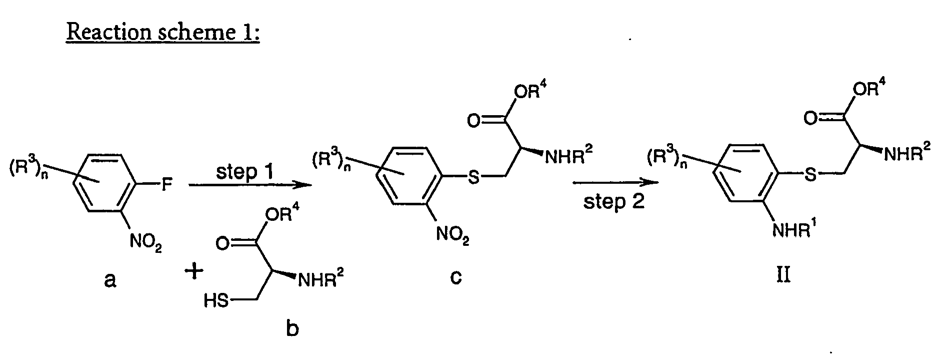 wherein R¹, R², R³ and R⁴ are as described for compounds of formula I and II.

In reaction scheme 1, substituted 2-nitro fluoro-aromatic of formula a) is reacted with protected cysteine (e.g. BOC-Cysteine-methylester) of formula b) to obtain the corresponding nitro-phenyl substituted protected cysteine of formula c). The reaction is conveniently carried out under basic conditions (with a diisopropylamine, e.g. ethyldiisopropylamine) in an appropriate organic solvent, such as hexane, diisopropylether, ethyl acetate, methanol, ethanol, propanol, dichloromethane, DMF or DMSO. The reaction temperature is preferably between -30°C to +150°C. Most preferred, the reaction is carried out with BOC-Cysteine-methylester in ethanol in the presence of ethyldiisopropylamine at a temperature of 110°C.

Compounds of formula a) and the protected cysteine (e.g. BOC-Cysteine-methylester) of formula b) are commercially available or synthesized according to methods known from textbooks about organic chemistry e.g. from J. March (1992), "Advanced Organic Chemistry: Reactions, Mechanisms, and Structure", 4^{th} ed. John Wiley & Sons).

The second step of the reaction is carried out in that the nitro group of the nitro-phenyl substituted protected cysteine of formula c) is reduced to the corresponding amino-phenyl substituted protected cysteine of formula II. The reduction reaction is carried out according to methods known in the art for example known in textbooks on organic chemistry e.g. J. March (1992), "Advanced Organic Chemistry: Reactions, Mechanisms, and Structure". 4^{th} ed. John Wiley & Sons). The reaction is conveniently carried out with a suitable reducing agent (e.g. Zinc and optional NH₄Cl) in acidic media with organic solvents such as hexane, diisopropylether, ethyl acetate, methanol, ethanol, propanol, dichloromethane, DMF, DMSO, preferably methanol. The reaction temperature is preferably between -30°C to +150°C.

The NH₂-group of compound of amino-phenyl substituted protected cysteine of formula II may be alkylated with R₁Hal, wherein R₁ is as defined above and Hal is chlorine or bromine.

The compounds of formula II-a and II-b (see below) are novel intermediates and therefore also subject of the present invention 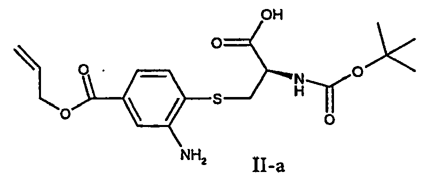 or

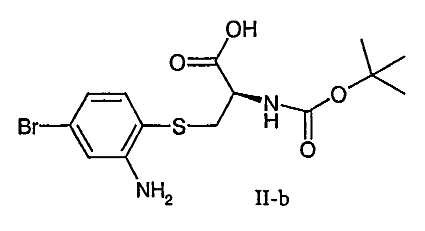

Compounds of the formula I are versatile building blocks for the synthesis of 1,5-benzothiazepines. Such benzothiazepine scaffolds have been used as a constrained dipeptide mimics in various enzyme inhibitors (protease, interleukin-1β-converting enzyme, elastase or angiotensin-converting enzyme, but also as GPCR antagonists (cholecystokinin, angiotensin II receptor). The possible different uses is described in G.C. Morton et al., Tet. Lett., 41 (2000) 3029-3033.

The benzothiazepines are prepared according reaction scheme 2: 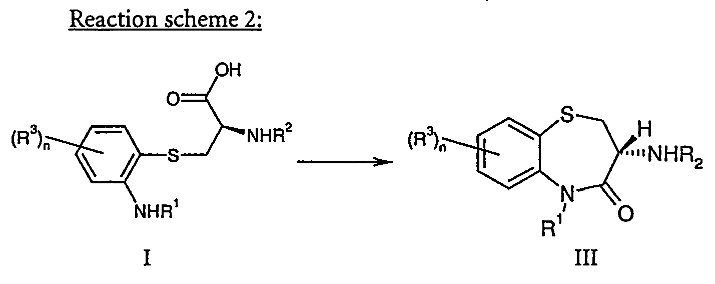 wherein R¹, R², R³, R⁴ and n are as described for compounds of formula I and II.

The cyclisation reaction of compound of formula I to obtain benzothiazepine of formula III is carried out thermally or in the presence of an appropriate reagent, for example as described in G.C. Morton et al., Tet. Lett., 41 (2000) 3029-3033). The reaction is conveniently carried out with a carbodiimide in an appropriate solvent such as hexane, xylene, diisopropylether, ethyl acetate, methanol, ethanol, propanol, dichloromethane, DMF or DMSO. The reaction temperature is preferably between -30°C to +150°C. The reaction is preferably carried out at room temperature in DMF in the presence of EDAC (1-ethyl-3-(3-dimethylaminopropyl) carbodiimide hydrochloride).

In the following examples the abbreviations used have the following significations.
- ISP-MS: ion spray positive mass spectroscopy
- ISN-MS: ion spray negative mass spectroscopy
- EI-MS: electron impact mass spectroscopy
- SFC: super critical fluid chromatography
- NMR: nuclear magnetic resonance spectroscopy
- IR: infra red spectroscopy
- HV: high vacuum
- min: minute(s)

### Example 1 (preparation of the starting material)

### (S)-3-(4-Bromo-2-nitro-phenylsulfanyl)-2-tert.-butoxycarbonylaino-propionic acid methyl ester

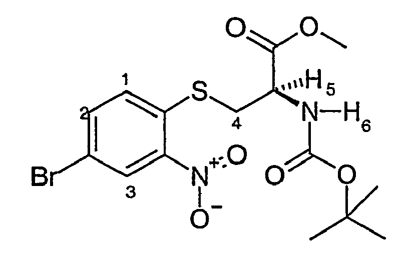

The reaction is carried out as described in M. K. Schwarz et al., J. Org. Chem. 1999, 64,2219-2231. To a solution of commercially available BOC-Cysteine-Methylester (27.38g, 116.35mmol, 1 eq),N-Ethyldiisopropylamine (DIPEA) (4.4mL, 25.75mmol, 2.5 eq) in EtOH (310 mL) was added 5-bromo-2-fluoronitrobenzene (25.62g ; 116,45mmol; 1 eq) and heated at 110°C for 3 h. The progress of the reaction was monitored by HPLC. The solvent was evaporated to a red-brown oil and this was partitioned between water (600mL) and ethyl acetate (3 X 300mL). Drying (Na₂SO₄), and evaporation gave crude product which was recrystallised with cyclohexane to give yellow crystals (38.75g, 77%). Analytical data: ¹H-NMR (CDCl₃, 400MHz): 1.430 (s, 9H, O(CH₃)); 3.350 (dd, 1H, H(4'), J_{4'-4''} =5.2Hz, J_{4'-5}=14.4Hz); 3.500 (dd,1H, H(4"), J_{4"-4'}=5.2Hz, J_{4"-5}=14.4Hz); 3.755 (s ,3H, OCH₃); 4.621 (m,1H, H(5)); 5.301 (d, 1H, H(6), J₆₋₅=6.4Hz); 7.449 (d, 1H, H(1), J₁₋₂=8.8Hz); 7.670 (dd,1H, H(2), J₁₋₂=8.8Hz, J₂₋₃=2.0Hz); 8.278 (d, 1H, H(3), J₂₋₃=2Hz). IR: 3353 cm⁻¹ (NH);1765 cm⁻¹ (Ester -C=O); 1686 cm⁻¹ (Carbamate -C=O); 1546 cm⁻¹(Amide -C=O); 1460 and 1329 cm⁻¹(NO2); 1220 cm⁻¹(Ester). MS: m/z=452.3 [M+NH₄⁺] with ⁷⁹Br; m/z=454.3 [M+ NH₄⁺] with ⁸¹Br; m/z=457.3 [M+Na⁺] with ⁷⁹Br; m/z=459.3 [M+Na⁺] with ⁸¹Br. Rf=0.38 in Ethyl acetate / Hexane 1 :2 on SiO₂.

### Example 2

### (S)-3-(2-Amino-4-bromo-phenylsulfanyl)-2-tert.-butoxycarbonylamino-propionic acid methyl ester

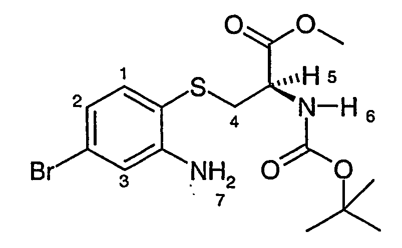

The reaction is carried out as described in J. Slade et.al., J. Med. Chem. 1985, 28, 1517-1521). A mixture of the nitro compound from above (40.34g, 92.6mmol, 1 eq), NH₄Cl (19.81g, 370.6mmol, 4 eq) is added and Zn (79.37g, 1.204mmol, 13 eq) in MeOH (950 mL) was heated at reflux for 16h and the resultant mixture filtered through Celite and washed with boiling MeOH. After concentration, the crude product was partitioned between ethyl acetate and a NaHCO₃ (aq.). The resultant oil was chromatographed using ethyl acetate / hexane / 3% triethylamine) to give the product (21.11g, 56%). Analytical data: ¹H-NMR (CDCl₃, 400MHz): 1.395 (s, 9H, O(CH₃)); 3.185 (d, 2H, H(4), J₄₋₅=4Hz) ; 3.617 (s, 3H, OCH₃); 4.446 (s, 2H, H(7)) ; 4.528 (m,1H, H(5)); 5.502 (d,1H, H(6), 1₆₋₅=8Hz) ; 6.793 (dd,1H, H(3), J₃₋₂=2Hz, J₃₋₁=8.4Hz) ;6.868 ( d,1H, H(3), J₃₋₂=2Hz), 7.225 (d,1H, H(1), J₁₋₂=8.4Hz). IR: 3356cm⁻¹ (-NH2, -NH) ; 1744cm⁻¹ (Ester -C=O); 1707cm⁻¹ (Carbamate -C=O) ; 1504cm⁻¹ (Amide) ; 1250cm⁻¹ (Ester). MS: m/z=405.3 [M+H⁺] with ⁷⁹Br; m/z=407.3 [M+H⁺] with ⁸¹Br. Rf=0.47 in ethyl acetate / hexane 1 :2 on SiO₂.

### Example 3.1 (large scale enzymatic hydrolysis)

### (S)-3-(2-Amino-4-bromo-phenylsulfanyl)-2-tert.-butoxycarbonylamino-propionic acid

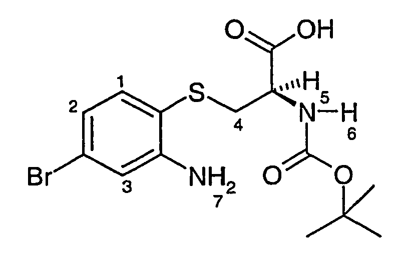

19.9g (48.02mmol) of N-tert.-butoxycarbonyl-3-(2-amino-4-bromophenylthio)-L-alanine methyl ester (97.8%) was dissolved in 750ml TBME and emulsified in 31 buffer solution (0.1M sodium chloride, 20mM sodium phosphate pH 7.5) under vigorous stirring. 12.0ml Alcalase 2.4 L and 30mg Substilisin A [both enzyme preparations are subtilisin Carlsberg from Novo Nordisk] were added and the pH was maintained at 7.5 under vigorous stirring by the controlled addition (pH-static) of 1.0N sodium hydroxide solution. After 7d 44.75ml of 1.0N sodium hydroxide solution was consumed and the conversion degree was >99% (HPLC analysis). The biphasic reaction mixture was separated. The aqueous phase was washed briefly with 11 TBME for the separation of small amounts of lipophilic impurities and traces of the remaining substrate. The combined organic phases were extracted with 1 x 250ml 0.1 M potassium phosphate buffer pH 7.6. The combined aqueous phases were acidified to pH 2 with 32% hydrochloric acid and extracted with 1.51 ethyl acetate. The resulting emulsion was separated by the addition of 10% Dicalite under stirring and a subsequent filtration. The aqueous phase was extracted with 2 x 11 ethyl acetate. The combined ethyl acetate phases were dried on anhydrous sodium sulfate and evaporated. The residue was dissolved in dichloromethane, evaporated and dried at HV to give 18.79g N-tert.-butoxycarbonyl-3-(2-amino-4-bromophenylthio)-L-alanine as a pale yellow solid (yield: 81.8%). Analytical data: ¹H-NMR (CDCl₃, 400MHz) 1.399 (s, 9H, OC(CH₃)); 3.184 (dd, 1H, SCH₂, J=14.2Hz, J=6.6Hz); 3.235 (dd, 1H, SCH₂, J=14.2Hz, J=6.6Hz); 4.515 (m, 1H, COCHNH); 5.517 (d, 1H, CONHCH, J=6.8Hz); 6.796 (dd, 1H, arom., J=2Hz, J=8.4Hz); 6.876 (d,1H, arom., J=2Hz); 7.250 (d, 1H, arom., J=8.4Hz). IR (ATR-IR) 3445 and 3355cm⁻¹ (-NH, -NH2); 2978 and 2929cm⁻¹ broad (-COOH); 1693cm⁻¹ (COOH-C=O,carbamate-C=O); 1508cm⁻¹ (amide-CO-NH); 1247 and 1157cm⁻¹ (COOH). MS (ESI-positive ionization) m/z=391.1 [M+H⁺] with ⁷⁹Br; m/z=393.1 [M+H⁺] with ⁸¹Br; m/z=413.2 [M+Na⁺] with ⁷⁹Br; m/z=415.2 [M+Na⁺] with ⁸¹Br. OR [α]_{D}= +53.3° (CHCl₃; c=1.0). HPLC analysis: column: ABZ+plus; mobile phase: A: 0.1% TFA in H₂O; B: MeCN; gradient B: 30 - 80% 0 -15 min, 80 - 30% 15-16 min, 30% 16-19.5 min; flow:1 ml/min; pressure: 50 - 80 bar; detection: UV, 300nm; retention times: 12.1 min (product-acid) 13.5 min (substrate-ester).

### Example 3.2 (small scale enzymatic hydrolysis)

### (S)-3-(2-Amino-4-bromo-phenylsulfanyl)-2-tert.-butoxycarbonylamino-propionic acid

1.65g (3.99mmol) of N-tert.-butoxycarbonyl-3-(2-amino-4-bromophenylthio)-L-alanine methyl ester (98%) was dissolved in 65ml TBME and emulsified in 240ml buffer solution (0.1M sodium chloride; 20mM sodium phosphate pH 7.5) under vigorous stirring. 0.75ml Alcalase 2.4 L [a subtilisin Carlsberg from Novo Nordisk] was added and the pH maintained at 7.5 under vigorous stirring by the controlled addition (pH-static) of 1.0N sodium hydroxide solution. After 33h 4.23ml of 1.0N sodium hydroxide solution was consumed and the conversion degree was >99% (HPLC analysis). The reaction mixture was acidified to pH 2 with 32% hydrochloric acid, filtered over Decalite. After phase separation the aqueous phase was extracted with 3 x 275ml ethyl acetate. The combined organic phases were dried on anhydrous sodium sulfate and evaporated. The residue was dissolved in dichloromethane, evaporated and dried at HV to give 1.56g N-tert.-butoxycarbonyl-3-(2-amino-4-bromophenylthio)-L-alanine as a pale yellow solid (yield: 99.8%).

### Example 3.3 (enzymatic hydrolysis with different solvents)

### (S)-3-(2-Amino-4-bromo-phenylsulfanyl)-2-tert.-butoxycarbonylamino-propionic acid

100mg (0.242mmol) of N-tert.-butoxycarbonyl-3-(2-amino-4-bromophenylthio)-L-alanine methyl ester (98%) was added to a system consisting of 20% organic co-solvent (see following table) and 80% buffer solution (0.1M sodium chloride; 3mM sodium phosphate pH 7.5) under vigorous stirring. 100ul Alcalase 2.4 L [a subtilisin Carlsberg from Novo Nordisk] was added and the pH maintained at 7.5 under vigorous stirring by the controlled addition (pH-static) of 0.1N sodium hydroxide solution. At the end of the reaction the conversion degree was determined by HPLC.

| organic co-solvent, total reaction volume | conversion^{a} in % | comments |
|---|---|---|
| ethyl acetate, 20ml | ~59% | Base consumption was exhausted (>200%) and the reaction was aborted. |
| THF, 50ml | >97% | 24mg (0.058mmmol) substrate and 50ul Alcalase |
| TBME, 25ml | >99% | |

| | | |
|---|---|---|
| ^{a}The conversion was calculated from the peak area of HPLC analysis at 300nm. | | |

### Example 3.4 (enzymatic hydrolysis with other enzymes)

### (S)-3-(2-Amino-4-bromo-phenylsulfanyl)-2-tert.-butoxycarbonylamino-propionic acid

200mg (0.493mmol) of N-tert.-butoxycarbonyl-3-(4-bromo-2-aminophenylthio)-L-alanine methyl ester (93.8%) was added to a system consisting of 5ml TBME and 20ml buffer solution (0.1M sodium chloride; 3mM sodium phosphate pH 7.0) under vigorous stirring. Enzyme [see following table] was added and the pH maintained at 7.0 under vigorous stirring by the controlled addition (pH-static) of 0.1N sodium hydroxide solution. After termination of the reaction the reaction mixture was washed twice with 25ml TBME, acidified to pH 2.5 with 25% HCl and extracted with 3 x 25ml ethyl acetate. The combined organic phases were dried over sodium sulfate, the solvent evaporated and the residue dried on a high vacuum.

| enzyme (company) | enzyme amount | time h | titrating agent consumed ml | product (amount; HPLC-purity) |
|---|---|---|---|---|
| Prozyme 6 | 42mg | 44 | 4.76 | 191mg; |
| (Amano Pharmaceuticals) | | | | 92.2% |
| Savinase 16L | 100µl | 43 | 4.969 | 195mg: |
| (Novo Nordisk) | | | | 90.7% |

### Example 4

### (S)-(7-Bromo-4-oxo-2,3,4,5-tetrahxdro-benzo[b][1,4]thiazepin-3-yl)-carbamic acid tert.-butyl ester

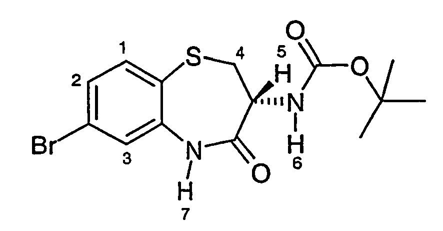

The reaction is carried out as described in G.C. Morton et al., Tet. Lett., 41 (2000) 3029-3033.). The product from the enzyme hydrolysis (15.2g, 38.8mmol, leq) and EDAC (1-Ethyl-3-(3-dimethylaminopropyl) carbodiimide hydrochloride) (7.44g, 38.8mmol, 1 eq) in DMF (500 mL) was stirred at room temperature for 16h, concentrated and the residue dissolved in ethyl acetate (500 ml) and extracted with aq. NaHCO₃ (1M) (500mL) and water (500mL). Drying (NaSO₄) and concentrated to give the product (14.5g, quantitative). Analytical data: ¹H-NMR (CDCl₃, 400MHz): 1.409 (s, 9H, O(CH₃)); 2.939 (t, 1H, H(4'), J=11.6Hz) ; 3.794 (dd, 1H, H(4"), J_{4"-5}=11Hz, J_{4"-4'}=6.4Hz); 4.439 (m,1H, H(5)) ; 5.567 (d,1H, H(6), J₆₋₅=8Hz) ; 7.306 (d,1H, H(2), J₂₋₁=8Hz, J₂₋₃=2Hz); 7.337 (d, 1H, H(3), J₃₋₂=2Hz); 7.748 (d,1H, H(1), J₁₋₂=8Hz); 8.042 (s,1H, H(7)). IR: 3284 and 3184 cm⁻¹ (-NH); 1729cm⁻¹ (Carbamate -C=O); 1678cm⁻¹ (Amide -C=O); 1574cm⁻¹ (Amide); 1251cm⁻¹ (Ester). MS: m/z=373.3 [M+H⁺] with ⁷⁹Br; m/z=375.3 [M+H⁺] with ⁸¹Br; m/z=395 [M+Na⁺] with ⁷⁹Br; m/z=397 [M+Na⁺] with ⁸¹Br. Rf=0.23 in Ethyl acetate /Hexane 1 :2 on SiO₂.

### Example 5

### (S)-4-(2-tert.-Butoxycarbonylamino-2-methoxycarbonyl-ethylsulfanyl)-3-nitro-benzoic acid allyl ester

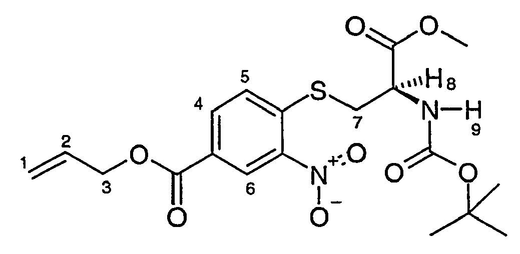

4-Fluoro-3-nitrobenzoic acid (49.95g, 269.8mmol, leq) was dissolved in methanol (350mL) and Cs₂CO₃ (44.06g,135.2mmol, 0.5eq) added and the solvent evaporated. Then the Cs-salt was dissolved in DMF, heated to 50°C and treated with allylbromide (22.8mL, 269.8mmol, leq). After 5 mins. the solvent was evaporated and the resultant solid triturated in Et₂O. Filtration and evaporation gave the crude allylester quantitatively. The next step is carried according to M. K. Schwarz et al., J. Org. Chem. 1999,64,2219-2231. The allyl ester (30g, 133.2mmol, leq), BOC-Cysteine-Methylester (31.35g, 133.2mmol, 1 eq) and DIPEA (68.3mL, 399.6mmol, 3eq) is added slowly (exothermic reaction is observed) in EtOH was stirred at rt for 3 hours, and concentrated to give a solid which was recrystallised from diisopropylether (46.16g, 79%). Analaytical data: ¹H-NMR (CDCl₃, 400MHz): 1.443 (s, 9H, O(CH₃));3.401 (dd,1H; H(7'), J_{7'-7"}=2.4Hz, J_{7'-8}=6Hz); 3.506 (dd, 1H, H(7"), J_{7"-7'}=2.4Hz, J_{7"-8}=6Hz); 3.777 (s ,3H, OCH₃); 4.608 (m, 1H, H(8)) ; 4.860 (dt, 2H, H(3',3"), J₃₋₂=5.6Hz ; J₃₋₁=1.6Hz); 5.322 (q,1H, H(1'), J=0.8Hz) ; 5.347 (t,1H, H(1"), J=0.8Hz) ; 5.406 (d,1H, H(9), J₉₋₈=1.6Hz); 6.032 (m, 1H, H(2)) ; 7.625 (d, 1H, H(5), J₅₋₄ =8.4Hz) ; 8.201 (dd,1H, H(4), J₄₋₅=8.4Hz, J₄₋₆=1.6Hz); 8.830 (d, 1H, H(6), J₆₋₄=1.6Hz). IR: 3350cm⁻¹(-NH) ; 1742cm⁻¹ (Ester-C=O); 1719cm⁻¹(Conj. Ester-C=O); 1686cm⁻¹ (Carbamate-C=O); 1523 and 1334cm⁻¹(-NO₂); 1245cm⁻¹(Ester). MS: m/z=441.3 [M+H⁺]; m/z=458.4 [M+ NH₄⁺]; m/z=463.2 [M+Na⁺].Rf=0.40 in Ethyl acetate / Hexane 1:2 on SiO₂.

### Example 6

### (S)-3-Amino-4-(2-tert.-butoxycarbonylamino-2-methoxycarbonyl-ethylsulfanyl)-benzoic acid allyl ester

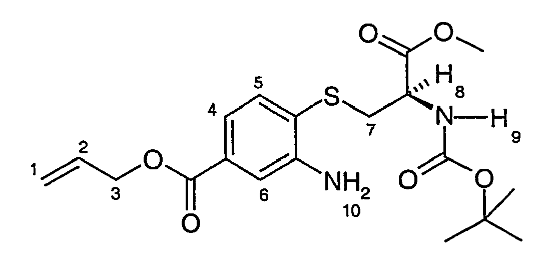

The reaction is carried out as described in J. Slade et.al., J. Med. Chem.1985, 28, 1517-1521. The above product (506mg, 1.15mmol, 1eq), aq. NH₄Cl (20mL) and Zn (976mg, 14.9mmol, 13eq) in DME (15mL) was stirred and heated at 80°C for 16h. The solvent was evaporated and the residue dissolved in ethyl acetate (250mL) and extracted with (aq.) sodium NaHCO₃ (1M) (3 X 50mL). The organic phase was dried with NaSO₄ and evaporated to give a yellow oil which crystallized after few days (276mg, 60%). Analytical data: ¹H-NMR (CDCl₃, 400MHz): 1.380 (s, 1H, O(CH₃)); 3.283 (d, 2H, H(7', 7"), J₇-₈=4.4Hz) ; 3.579 (s,3H, OCH₃); 4.445 (s, 2H, H(10',10")) ; 4.554 (m,1H, H(8)) ; 4.787 (ddd, 2H, H(2', 2"), J=1.2Hz) ; 5.283 (dd,1H, H(1'), J_{1'-2}=10.6Hz, J_{1'-1"}=1.2Hz) ; 5.390 (dd, 1H, H(1"), J_{1"-2}=17.8Hz, J_{1"-1}=1.2Hz) ; 5.506 (d, 1H, H(9), J₉₋₈=7.6Hz) ; 6.015 (m, 1H, H(8)); 7.360 (dd, 1H, H(2), J₄₋₅=8Hz, J₄₋₆=1.6Hz); 7.398 (d, 1H, H(6), J₆₋₄ =1.6Hz) ; 7.425 (d,1H, H(5), J₅₋₄=8Hz). IR: 3378cm⁻¹(-NH, -NH₂); 1751cm⁻¹(Ester - C=O); 1713cm⁻¹(Conj. Ester -C=O) ; 1685cm⁻¹(Carbamate-C=O) ; 1511cm⁻¹(-Amide) ; 1220cm⁻¹(Ester). MS: m/z=411.3 [M+H⁺]; m/z=433.3 [M+Na⁺].Rf=0.27 in Ethyl acetate / Hexane 1 :2 on SiO₂.

### Example 7.1 (large scale enzymatic hydrolysis)

### (S)-3-Amino-4-(2-tert.-butoxycarbonylamino-2-carboxy-ethylsulfanyl)-benzoic acid allyl ester

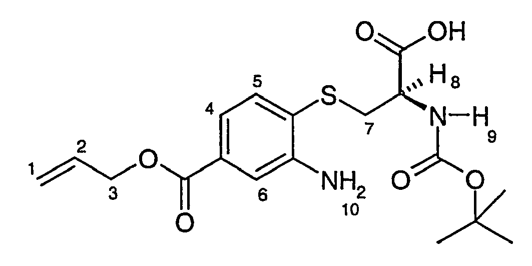

6.4g (15.658mmol) of N-tert.-butoxycarbonyl-3-(4-alloxycarbonyl-2-aminophenylthio)-L-alanine methyl ester (99%) was dissolved in 480ml TBME and emulsified in 1.51 buffer solution (0.1M sodium chloride;160mM sodium phosphate pH 7.5) under vigorous stirring. 12.0ml Alcalase 2.4 L [a subtilisin Carlsberg from Novo Nordisk] was added and the pH maintained at 7.5 under vigorous stirring by the controlled addition (pH-static) of 1.0N sodium hydroxide solution. After 48.5h 16.6ml of 1.0N sodium hydroxide solution was consumed and the conversion degree was >97% (HPLC analysis). After phase separation the aqueous phase was extracted once with 1l TBME. The combined TBME phases were extracted with 2 x 0.410.1 M potassium phosphate buffer pH 7.6. The combined aqueous phases were acidified to pH 2 with 32% hydrochloric acid and extracted with 3 x 0.51 ethyl acetate. In case a stabile emulsion was formed the phase separation was achieved by filtration on Dicalite. The combined ethyl acetate phases were dried on anhydrous sodium sulfate and evaporated. The residue was dissolved in dichloromethane, evaporated and dried at HV to give 5.85g N-tert.-butoxycarbonyl-3-(4-alloxycarbonyl-2-aminophenylthio)-L-alanine as a yellow highly viscous oil (yield: 94.6%). Analyticat data: ¹H-NMR (DMSO, 400MHz) 1.338 (s, 9H, OC(CH₃)); 3.077 (dd,1H, SCH₂, J=12.8Hz, J=6.8Hz); 3.238 (dd,1H, SCH₂, J=13.8Hz, J=4.6Hz); 3.867 (m,1H, COCHNH); 4.753 (d, 2H, COOCH₂, J=5.2Hz); 5.526 (d,1H, CH=CH₂, J=10Hz); 5.538 (d,1H, CH=CH₂, J=17.6Hz); 5.595 (m, 2H, NH₂); 6.018(ddtr, 1H, CH₂CH=CH₂, J=16.6Hz, J=10.6Hz, J=5.6Hz); 6.519 (m, 1H, CONHCH); 6.876 (d, 1H, arom. CSCH, J=8.4Hz); 7.330 (d, 1H, arom. CSCHCH, J=8.4Hz); 7.338 (s,1H, arom. CHCNH₂). IR (ATR-IR) 3420 and 3355cm⁻¹ (-NH, -NH2); 2980 and 2935 cm⁻¹ broad (-COOH); 1705cm⁻¹ (COOH -C=O, carbamate -C=O); 1510cm⁻¹ (amide-CO-NH); 1486cm⁻¹ aromate; 1229 and 1160cm⁻¹ (COOH); 982 and 932 cm⁻¹ (vinyl, -C=CH₂). MS (ESI-positive ionisation) m/z=397.1 [M+H⁺]; m/z=419.4 [M+Na⁺]. OR [α]_{D}= +16.16 ° (CHCl₃; c=1.0). HPLC analysis: column: ABZ+plus; mobile phase: A: 0.1% TFA in H₂0; B: MeCN; gradient B: 30 - 80% 0 -15 min, 80 - 30% 15 - 16 min, 30% 16 -19.5 min; flow: 1 ml/min; pressure:50 - 80 bar; detection: UV, 300nm; retention times: 11.2 min (product-acid); 12.7 min (substrate-ester).

### Example 7.2 (small scale enzymatic hydrolysis)

### (S)-3-Amino-4-(2-tert.-butoxycarbonylamino-2-carboxy-ethylsulfanyl)-benzoic acid allyl ester

0.769mg (1.658mmol) of N-tert.-butoxycarbonyl-3-(4-alloxycarbonyl-2-aminophenyl-thio)-L-alanine methyl ester was dissolved in 40ml TBME and emulsified in 220ml 0.1M sodium chloride solution; 3mM sodium phosphate buffer pH 7.5 under vigorous stirring. 1.0ml Alcalase 2.4 L [a subtilisin Carlsberg from Novo Nordisk] was added and the pH maintained at 7.5 under vigorous stirring by the controlled addition (pH-static) of 1.0N sodium hydroxide solution. After 45.4h 23.6ml of 1.0N sodium hydroxide solution was consumed and the conversion degree was > 97% (HPLC analysis). The reaction mixture was extracted with 100ml TBME. After phase separation the aqueous phase was acidified to pH 2.3 with 32% hydrochloric acid and extracted with 3 x 250ml ethyl acetate. In case a stabile emulsion was formed the phase separation was achieved by filtration on Dicalite. The combined ethyl acetate extracts were dried on anhydrous sodium sulfate and evaporated. The residue was dissolved in dichloromethane, evaporated and dried at HV to give 604mg N- tert.-butoxycarbonyl-3-(4-alloxycarbonyl-2-aminophenylthio)-L-alanine as a pale yellow solid (yield: 93.9%, purity: 95%).

### Example 7.3 (enzymatic hydrolysis with different solvents)

### (S)-3-Amino-4-(2-tert.-butoxycarbonylamino-2-carboxy-ethylsulfanyl)-benzoic acid allyl ester

200mg (0.487mmol) of N-tert.-butoxycarbonyl-3-(4-alloxycarbonyl-2-aminophenylthio)-L-alanine methyl ester (96.7%) was added to a system consisting of 2 to 5ml of an organic co-solvent (see following table) and 20ml buffer solution (0.1M sodium chloride; 3mM sodium phosphate pH 7.0) under vigorous stirring. Alcalase 2.4 L [a subtilisin Carlsberg from Novo Nordisk, amount see following table] was added and the pH maintained at 7.0 under vigorous stirring by the controlled addition (pH-static) of 0.1N sodium hydroxide solution. The reaction was monitored by the consumption of titrating agent, and the formation of the product confirmed by HPLC.

| organic co-solvent, amount | Alcalase, amount | time h | comments |
|---|---|---|---|
| ethanol, 2.0ml | 100µl | 72 | consumption of titrating agent at the end of the reaction: 115% |
| acetone, 5.0ml | 200µl | 171 | consumption of titrating agent at the end of the reaction: 103% |
| THF, 5.0ml | 100µl | 146 | consumption of titrating agent at the end of the reaction: 98% |
| TBME, 5.0ml | 100µl | 72 | consumption of titrating agent at the end of the reaction: 119% |

### Example 7.4 (enzymatic hydrolysis with other enzymes)

### (S)-3-Amino-4-(2-tert.-butoxycarbonylamino-2-carboxy-ethylsulfanyl)-benzoic acid allyl ester

200mg (0.487mmol) of N-tert.-butoxycarbonyl-3-(4-alloxycarbonyl-2-aminophenylthio)-L-alanine methyl ester (96.7%) was added to a system consisting of 5ml TBME and 20ml buffer solution (0.1M sodium chloride; 3mM sodium phosphate pH 7.0) under vigorous stirring. Enzyme [see following table] was added and the pH maintained at 7.0 under vigorous stirring by the controlled addition (pH-static) of 0.1N sodium hydroxide solution. The reaction was monitored by the consumption of titrating agent, and the formation of the product confirmed by HPLC.

| enzyme (company) | enzyme amount | time h | comments |
|---|---|---|---|
| Prozyme 6 (Amano Pharmaceuticals) | 50mg | 42 | consumption of titrating agent at the end of the reaction: 112% |
| Proteinase K (Fluka) | 25mg | 137 | slow: after 137h only 40% (of theory) of titrating agent was consumed |

### Example 7.5 (enzymatic hydrolysis at higher temperature)

### (S)-3-Amino-4-(2-tert.-butoxycarbonylamino-2-carboxy-ethylsulfanyl)-benzoic acid allyl ester

200mg (0.487mmol) of N-tert.-butoxycarbonyl-3-(4-alloxycarbonyl-2-aminophenylthio)-L-alanine methyl ester (96.7%) was added to a system consisting of 5ml TBME and 20ml buffer solution (0.1M sodium chloride; 3mM sodium phosphate pH 7.0) under vigorous stirring at 40°C.100µl of Alcalase 2.4 L [a subtilisin Carlsberg from Novo Nordisk] was added and the pH maintained at 7.0 under vigorous stirring at 40°C by the controlled addition (pH-static) of 0.1N sodium hydroxide solution. After a consumption 5.44ml of titrating agent (112% of theory; after 17.4h) the reaction mixture was washed with 25ml TBME, acidified to pH 2.5 with 1N HCl and extracted with 3 x 25ml ethyl acetate. The combined organic phases were dried over sodium sulfate, the solvent evaporated and the residue dried on a high vacuum to give 190mg (0.479mmol; 98%) of the product acid in 94.5% HPLC-purity (area-%).

### Example 8

### (S)-3-tert.-Butoxycarbonylamino-4-oxo-2,3,4,5-tetrahydro-benzo[b][1,4]thiazepine-7-carboxylic acid allyl ester

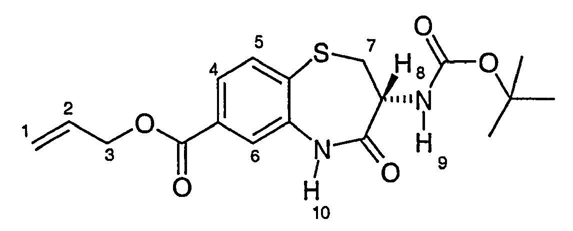

The reaction is carried out as described in G.C. Morton et al., Tet. Lett., 41 (2000) 3029-3033. The product from the enzyme hydrolysis (5.6g, 14.1mmol, leq) was dissolved in DMF (75mL) and treated with EDAC (2.70g, 14.1mmol, leq) and stirred at room temperature for 16h. After concentration the residue was dissolved in ethyl acetate (500mL) and extracted with (aq.) NaHCO₃ (1M) (3 X 150mL) and water (3 X 150mL). The organic phase was dried (Na₂SO₄) and evaporated to give yellow oil. After chromatography with ethyl acetate / hexane 1 :2. yellow crystals were obtained (4.96g, 93%). Analytical data: ¹H-NMR (CDCl₃, 400MHz):1.400 (s,1H, O(CH₃)); 3.014 (t,1H, H(7'or 7"), J=12Hz) ; 3.815 (dd,1H, H(7'or 7"), J₇₋₈=12Hz, J_{7'-7"}=6.4Hz) ; 4.466 (m,1H, H(8)) ; 4.835 (d, 2H, H(3', 3"), J₃₋₂= 5.7Hz ) ; 5.316 (dd,1H, H(1'), J_{1'-2}=10.5Hz, J_{1'- 1"}=1.2Hz) ; 5.413 (dd,1H, H(1"), J_{1"-2}=17.2Hz, J_{1"-1}=1.2Hz) ; 5.581 (d,1H, H(9), J_{9- 8}=8Hz ) ; 6.013 (m, 1H, H(8)) ; 7.338 (d, 1H, H(5), J₅₋₄=11.1Hz); 7.704 (s, 1H, H(10)); 7.751 (d,1H, H(6), J₆₋₄=1.8Hz) ;7.338 (dd, 1H, H(2), J₄₋₅=11.1Hz, J₄₋₆=1.8Hz). IR: 3206cm^{- 1} (-NH); 1720cm⁻¹ (Carbamate -C=O); 1671cm⁻¹ (Amide-C=O); 1500cm⁻¹ (Amide-CO-NH) ; 1209cm⁻¹ (Ester). MS: m/z=379.3 [M+H⁺];m/z=401.4 [M+Na⁺]. Rf=0.36 in Ethyl acetate / Hexane 1 :2 on SiO₂.

### Example 9

### (S)-3-Amino-4-(2-tert.-butoxycarbonylamino-2-carboxy-ethylsulfanyl)-benzoic acid

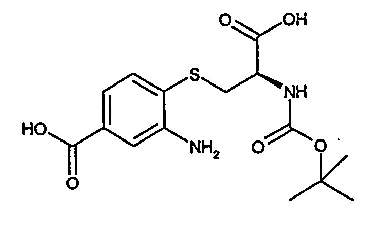

200mg (0.540mmol) of N-tert.-butoxycarbonyl-3-(4-carboxy-2-aminophenylthio)-L-alanine methyl ester was added to a system consisting of 5ml TBME and 20ml buffer solution (0.1M sodium chloride; 3mM sodium phosphate pH 7.0) under vigorous stirring. 100µl of Alcalase 2.5 L [a subtilisin Carlsberg from Novo Nordisk]was added and the pH maintained at 7.0 under vigorous stirring by the controlled addition (pH-static) of 0.1N sodium hydroxide solution. After a consumption 5.847ml of titrating agent (108% of theory; after 1.5h) the reaction mixture was washed twice with 25ml TBME, acidified to pH 2.5 with 25% HCl and extracted with 3 x 25ml ethyl acetate. The combined organic phases were dried over sodium sulfate, the solvent evaporated and the residue dried on a HV to give 187mg (0.479mmol) N-tert.-butoxycarbonyl-3-(2-amino-4-carboxyphenylthio)-L-alanine as yellow crystals (yield: 93.4%). Analytical data: HPLC-purity: 96.4%% (area). ¹H-NMR (DMSO, 400MHz) 1.378 (s, 9H, OC(CH₃)); 2.982 (dd, 1H, SCH₂, J=13.2Hz, J=9.6Hz); 3.150 (dd,1H, SCH₂, J=13.2Hz, J=4.4Hz); 3.950 (m,1H, COCHNH); 5.542 (m, 2H, -NH₂); 7.067 (dd,1H, CONHCH, J=8.0Hz, J=1.6Hz); 7.201(d, 1H, arom. SCCH, J=8.0Hz); 7.298 (d,1H, arom. SCCHCH, J=8.0Hz); 7.323 (d,1H, arom. CHCNH₂, J=1.6Hz);12.73 (bs, 2H, 2 -COOH). IR: 3445 and 3347cm⁻¹ (-NH, -NH2); 2977 and 2930cm⁻¹ broad (-COOH);1720 and 1692cm⁻¹ (COOH -C=O, carbamate -C=O); 1608cm⁻¹ (-COO⁻); 1509cm⁻¹ (amide -CO-NH);1486 cm⁻¹ (aromate); 1247 and 1163cm⁻¹ (COOH). ISN-MS: m/z=355.1 [M-H⁺]. OR [α]_{D}= +13.00° (EtOH; c=1.0).

## Claims

1. A process for the preparation of compounds of formula I 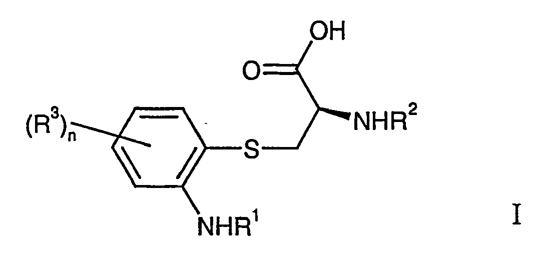 wherein
R¹ is hydrogen or alkyl;
R² is an amino protecting group;
each R³ is independently halogen, carboxyl, alkoxycarbonyl, alkenyloxycarbonyl or benzyloxycarbonyl;
n is 1 or 2,
which process comprises
reacting compounds of formula II 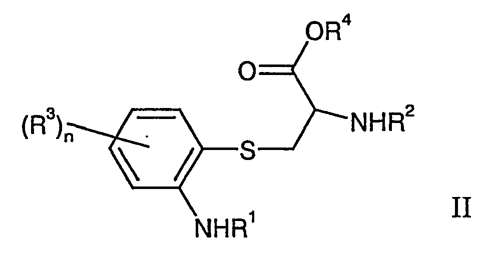 wherein R¹, R², R³ and n are as defined above and
R⁴ is alkyl or benzyl,
with a protease in an aqueous system containing an organic co-solvent.

2. A process according to claim 1, **characterized in that** compound of formula II is 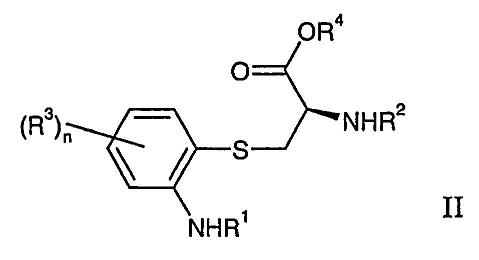 wherein R¹, R², R³, R⁴ and n are as defined in claim 1.

3. A process according to claims 1 and 2, **characterized in that**
R¹ is hydrogen or alkyl;
R² is an amino protecting group;
each R³ is independently halogen, carboxyl or alkenyloxycarbonyl;
R⁴ is alkyl or benzyl;
n is 1 or 2.

4. A process according to any one of claims 1-3, **characterized in that**
R¹ is hydrogen;
R² is an amino protecting group;
R³ is halogen, carboxyl or alkenyloxycarbonyl;
R⁴ is alkyl;
n is 1.

5. A process according to any one of claims 1-4, **characterized in that** the reaction is carried out at pH of 6.0 - 8.5.

6. A process according to any one of claims 1-5, **characterized in that** the reaction is carried out with a Bacillus protease, subtilisin, an Aspergillus protease or a Tritachium protease.

7. A process according to any one of claims 1-6, **characterized in that** the organic co-solvent is tetrahydrofuran, dioxan, tert.-butyl methyl ether, a C₁₋₈-alkohol, ethyl acetate, dimethylsulfoxide, dimethylacetamide or acetone.

8. Compound of formula 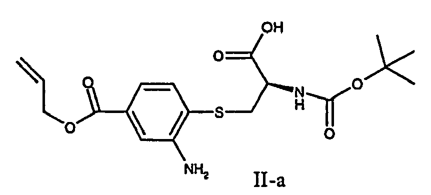 or

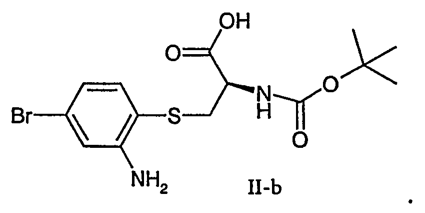

9. Process according to any one of claims 1-7, **characterised in that** the compound of formula I is further processed to a benzothiazepine of formula III 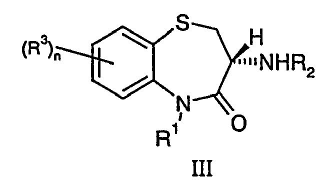 wherein R¹, R², R³, R⁴ and n are as described for compounds of formula I and II.

## Patentansprüche

1. Verfahren zur Herstellung von Verbindungen der Formel I 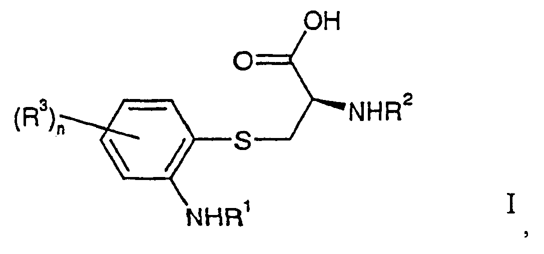 worin
R¹ Wasserstoff oder Alkyl ist;
R² eine Aminoschutzgruppe ist;
jedes R³ unabhängig voneinander Halogen, Carboxyl, Alkoxycarbonyl, Alkenyloxycarbonyl oder Benzyloxycarbonyl ist;
n 1 oder 2 ist,
wobei das Verfahren
das Umsetzen von Verbindungen der Formel II 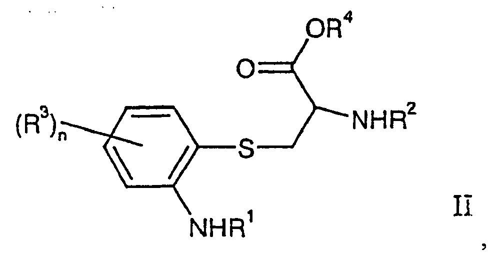 worin R¹, R², R³ und n wie oben definiert sind und
R⁴ Alkyl oder Benzyl ist,
mit einer Protease in einem wässerigen System, enthaltend ein organisches Hilfslösungsmittel, umfaßt.

2. Verfahren nach Anspruch 1, **dadurch gekennzeichnet, daß** die Verbindung der Formel II 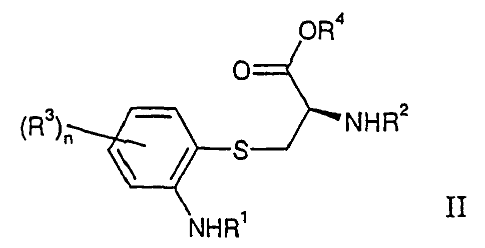 ist, worin R¹, R², R³, R⁴ und n wie in Anspruch 1 definiert sind.

3. Verfahren nach den Ansprüchen 1 und 2, **dadurch gekennzeichnet, daß**
R¹ Wasserstoff oder Alkyl ist;
R² eine Aminoschutzgruppe ist;
jedes R³ unabhängig voneinander Halogen, Carboxyl oder Alkenyloxycarbonyl ist;
R⁴ Alkyl oder Benzyl ist;
n 1 oder 2 ist.

4. Verfahren nach einem der Ansprüche 1 bis 3, **dadurch gekennzeichnet, daß**
R¹ Wasserstoff ist;
R² eine Aminoschutzgruppe ist;
R³ Halogen, Carboxyl oder Alkenyloxycarbonyl ist;
R⁴ Alkyl ist;
n 1 ist.

5. Verfahren nach einem der Ansprüche 1 bis 4, **dadurch gekennzeichnet, daß** die Reaktion bei einem pH von 6,0 bis 8,5 durchgeführt wird.

6. Verfahren nach einem der Ansprüche 1 bis 5, **dadurch gekennzeichnet, daß** die Reaktion mit einer Bacillus-Protease, Subtilisin, einer Aspergillus-Protease oder einer Tritachium-Protease durchgeführt wird.

7. Verfahren nach einem der Ansprüche 1 bis 6, **dadurch gekennzeichnet, daß** das organische Hilfslösungsmittel Tetrahydrofuran, Dioxan, tert-Butylmethylether, ein C₁₋₈-Alkohol, Ethylacetat, Dimethylsulfoxid, Dimethylacetamid oder Aceton ist.

8. Verbindung der Formel 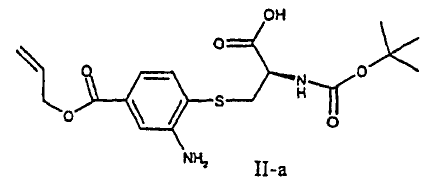 oder

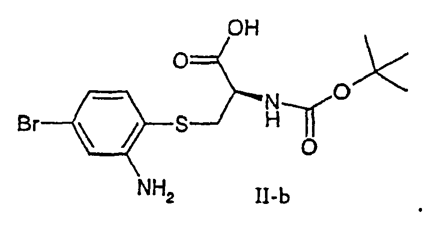

9. Verfahren nach einem der Ansprüche 1 bis 7, **dadurch gekennzeichnet, daß** die Verbindung der Formel I weiter zu einem Benzothiazepin der Formel III 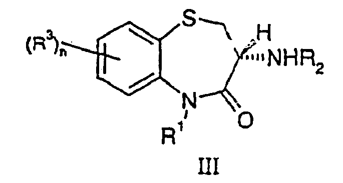 verarbeitet wird, worin R¹, R², R³, R⁴ und n wie für die Verbindungen der Formel I und II beschrieben sind.

## Revendications

1. Procédé de préparation de composés de formule I 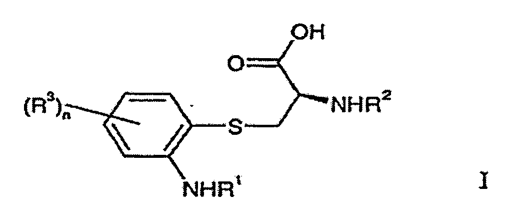 dans laquelle
R¹ est un hydrogène ou un alkyle ;
R² est un groupe amino protecteur ;
chaque R³ est indépendamment un halogène, un carboxyle, un alcoxycarbonyle, un alcènyloxycarbonyle ou un benzyloxycarbonyle ;
n est 1 ou 2,
ledit procédé comprend
la réaction de composés de formule II 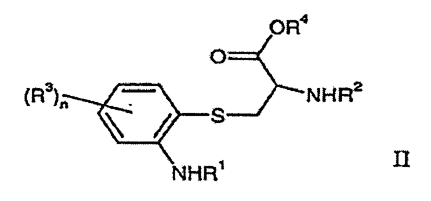 dans laquelle R¹, R², R³ et n sont tels que définis ci-dessus et
R⁴ est un alkyle ou un benzyle,
avec une protéase dans un système aqueux contenant un co-solvant organique.

2. Procédé selon la revendication 1, **caractérisé en ce que** le composé de formule II est 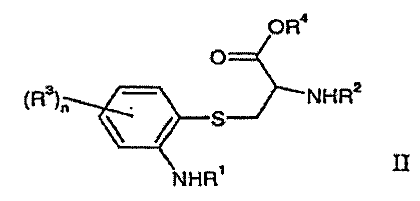 dans laquelle R¹, R², R³, R⁴ et n sont tels que définis dans la revendication 1.

3. Procédé selon les revendications 1 et 2, **caractérisé en ce que**
R¹ est un hydrogène ou un alkyle ;
R² est un groupe amino protecteur ;
chaque R³ est indépendamment un halogène, un carboxyle ou un alcènyloxycarbonyle ;
R⁴ est un alkyle ou un benzyle ;
n est 1 ou 2.

4. Procédé selon l'une quelconque des revendications 1 à 3, **caractérisé en ce que**
R¹ est un hydrogène ;
R² est un groupe amino protecteur ;
R³ est un halogène, un carboxyle ou un alcènyloxycarbonyle ;
R⁴ est un alkyle ;
n est 1.

5. Procédé selon l'une quelconque des revendications 1 à 4, **caractérisé en ce que** la réaction est menée à un pH de 6,0-8,5.

6. Procédé selon l'une quelconque des revendications 1 à 5, **caractérisé en ce que** la réaction est menée avec une protéase de Bacillus, la subtilisine, une protéase d'Aspergillus ou une protéase de Tritachium.

7. Procédé selon l'une quelconque des revendications 1 à 6, **caractérisé en ce que** le co-solvant organique est le.tétrahydrofuranne, le dioxanne, le tert-butyl méthyl éther, un alcool en C₁ à C₈, l'acétate d'éthyle, le diméthylsulfoxyde, le diméthylacétamide ou l'acétone.

8. Composé de formule 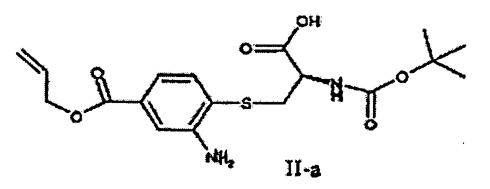 ou

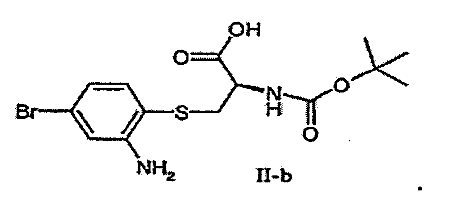

9. Procédé selon l'un quelconque des revendications 1 à 7, **caractérisé en ce que** le composé de formule I est en outre transformé en benzothiazépine de formule III 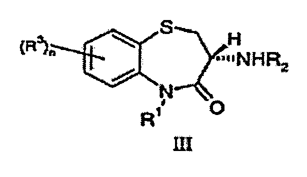 dans laquelle R¹, R², R³, R⁴ et n sont tels que décrits pour les composés de formule I et II.
